(19) Europäisches Patentamt
European Patent Office
Office européen des brevets

(11) EP 3 044 349 B1

(12) FASCICULE DE BREVET EUROPEEN

(45) Date de publication et mention de la délivrance du brevet:
**27.03.2019 Bulletin 2019/13**

(21) Numéro de dépôt: **14766155.7**

(22) Date de dépôt: **11.09.2014**

(51) Int Cl.:
***C25B 1/00*** $^{(2006.01)}$ ***C12P 3/00*** $^{(2006.01)}$
***C01B 3/04*** $^{(2006.01)}$ ***C01B 13/02*** $^{(2006.01)}$
***C25B 1/04*** $^{(2006.01)}$ ***H01M 8/16*** $^{(2006.01)}$

(86) Numéro de dépôt international:
**PCT/EP2014/069418**

(87) Numéro de publication internationale:
**WO 2015/036496 (19.03.2015 Gazette 2015/11)**

(54) **PROCÉDÉ DE DISSOCIATION DE L'EAU À BASE DU PHOTOSYSTÈME II (PSII)**

WASSERSPALTUNG PROZESS MIT PHOTOSYSTEM II (PSII)

PROCESS FOR WATER DISSOCIATION BASED ON PHOTOSYSTEM II (PSII)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.09.2013 BE 201300598**

(43) Date de publication de la demande:
**20.07.2016 Bulletin 2016/29**

(73) Titulaire: **H2Win**
**1400 Nivelles (BE)**

(72) Inventeur: **LORGE, Philippe**
**B-1400 Nivelles (BE)**

(74) Mandataire: **Gevers Patents**
**Intellectual Property House**
**Holidaystraat 5**
**1831 Diegem (BE)**

(56) Documents cités:
**EP-A2- 1 134 585 US-A1- 2010 200 049**

- **DMITRIY SHEVELA ET AL: "Probing the turnover efficiency of photosystem II membrane fragments with different electron acceptors", BIOCHIMICA ET BIOPHYSICA ACTA. BIOENERGETICS, AMSTERDAM, NL, vol. 1817, no. 8, 29 mars 2012 (2012-03-29), pages 1208-1212, XP028430079, ISSN: 0005-2728, DOI: 10.1016/J.BBABIO.2012.03.038 [extrait le 2012-04-06]**
- **JURSINIC P A ET AL: "Enhanced oxygen yields caused by double turnovers of Photosystem II induced by dichlorobenzoquinone", BIOCHIMICA ET BIOPHYSICA ACTA. BIOENERGETICS, AMSTERDAM, NL, vol. 934, no. 2, 6 juillet 1988 (1988-07-06), pages 177-185, XP023350243, ISSN: 0005-2728, DOI: 10.1016/0005-2728(88)90180-6 [extrait le 1988-07-06]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 044 349 B1

## Description

La présente invention se rapporte à un procédé pour dissocier une solution aqueuse comprenant :

- une première oxydation électrochimique de la solution aqueuse en présence de lumière puisée, laquelle lumière pulsée est générée à partir d'une première source d'énergie lumineuse à une valeur de fréquence de pulsion de pulsion prédéterminée, par une composition enzymatique à base d'un premier complexe enzymatique PSII, isolé d'un deuxième complexe enzymatique PSI, avec production d'oxygène, d'électrons libres et de protons libres dans la solution aqueuse,
- une capture desdits électrons libres et desdits protons libres, et
- une capture séparée d'oxygène.

Eventuellement, la lumière pulsée est une lumière cohérente, c'est-à-dire générée par un laser.
Un tel procédé est connu par exemple du document Biochimica et Biophysica Acta 1817 (2012) 1028-1212, de Sheleva et Messinger, qui divulgue l'oxydation électrochimique de l'eau en présence de lumière.
Ce procédé trouve tout particulièrement son application dans le cadre de la production d'énergie renouvelable à partir de la lumière solaire.
Le PSII est un complexe moléculaire enzymatique photosensible comprenant des pigments (chlorophylle) qui est le siège de l'hydrolyse de l'eau dans les chloroplastes présent dans le cytoplasme de cellules végétales selon la réaction suivante :

$$2H_2O \xrightarrow{h\nu,\ PSII} O_2 + 2H^+ + 2e-$$

, où hv correspond à un photon de lumière, par exemple de lumière solaire, $H_2O$ est l'eau, $H^+$ représente un proton libre et e-représente un électron libre.
Le complexe enzymatique PSII, de par sa fonction d'hydrolyseur, produit, à partir de lumière, d'une part de l'oxygène, et, d'autre part, des électrons libres et des protons libres est d'un intérêt particulier dans le cadre de son utilisation pour la production, par l'intermédiaire d'une cathode, d'un carburant propre qu'est l'hydrogène gazeux (sous forme de gaz sous conditions standards de température et de pression). En effet, la cathode permet de recombiner les e- et les $H^+$ (réaction de réduction des protons) pour former de l'hydrogène. C'est dans cette optique que ce complexe enzymatique est un acteur prometteur de production d'un carburant propre, c'est-à-dire dont la combustion n'est pas associée à la production de $CO_2$, à partir d'énergie naturelle : la lumière, qui de surcroît, constitue une source d'énergie quasi-inépuisable.
Dès lors, l'hydrogène gazeux, résultant de la réduction des protons libres qui s'associent aux électrons libres, se combine à l'oxygène résultant de la réaction d'hydrolyse afin de réaliser une réaction de combustion qui résulte en une production, d'une part, d'énergie et, d'autre part, d'eau qui peut être à nouveau hydrolysée par le complexe PSII, de sorte qu'un cycle de l'eau est formé, constituant ainsi une source quasi-inépuisable de carburant propre. De plus, l'oxydation assistée par le complexe PSII permet la formation d'oxygène et d'hydrogène *in situ,* et répond à une question actuellement problématique de conditionnement de l'hydrogène qui, puisqu'il est formé *in situ,* ne nécessite plus d'être stockés sous des formes conditionnement contraignantes telles que celles connues actuellement : volumes importants, pressions importantes, etc.
Selon le document Biochimica et Biophysica Acta 1817 (2012) 1028-1212*,* il y a absorptions successives de photons de lumière par les pigments du PSII. A chaque absorption correspond une réaction chimique d'oxydoréduction qui prend place dans le complexe enzymatique PSII. Il est par ailleurs connu de l'état de la technique que, de manière exclusive, l'absorption successive de quatre photons de lumière par les pigments du PSII autorise la réalisation successive de quatre réactions d'oxydoréduction formant un cycle photochimique (cycle de Kok) à l'origine de l'oxydation de l'eau en oxygène, d'une part, et en protons libres et électrons libres, d'autre part.
Par ailleurs, il est connu de l'état de la technique que l'activité du complexe PSII est entre autre régulée par celle du complexe PSI.
L'activité du PSII est mesurée par la vitesse de production d'oxygène dans l'eau, plus cette vitesse est élevée, plus le rendement de la réaction d'oxydation de l'eau par le PSII est élevé, c'est-à-dire plus la quantité en oxygène produite est élevée.
Il est en effet connu que, la présence du complexe PSI, de part sa fonction dans le mécanisme de production d'énergie par photosynthèse, consomme les protons et électrons libres destinés indirectement par la suite à la réaction de synthèse des sucres par polymérisation du $CO_2$.
Il est donc avantageux que le complexe PSII soit isolé du complexe PSI pour que la réaction d'oxydation de

l'eau augmente en rendement. En pratique, le complexe PSII est isolé du système PSI, ce dernier étant remplacé par un capteur (dispositif, produit, matériau) ou un groupe de capteurs d'électrons et de protons qui présente la propriété de ne pas inhiber l'activité du PSII. A titre exemplatif, le capteur d'électrons et de protons peut aussi être une cathode permettant la réduction des protons en hydrogène gazeux.

Par le terme « isolé », on entend au sens de l'invention que le complexe PSI n'est pas en mesure de coopérer avec le complexe enzymatique PSII.

Dans le procédé susmentionné, l'activité du PSII est principalement limitée par la concentration et la nature des capteurs d'électrons et des capteurs de protons ou des capteurs d'électrons et de protons qui, par exemple, réagissent avec les protons libres et les électrons libres pour subir une réduction.

Cet aspect est d'ailleurs souligné dans l'article de Sheleva et Messinger mentionné au début et dans lequel les auteurs démontrent l'efficacité des turnovers pour un complexe enzymatique PSII en fonction de la nature chimique des capteurs d'électrons.

Par le terme « turnovers », il faut entendre le nombre d'étape de chaque cycle de Kok bouclé par unité de temps, étant entendu qu'un cycle de Kok complet comprend quatre turnovers successifs puisque associé à l'absorption consécutive de quatre photons de lumière.

Lors de l'interaction de la lumière avec le complexe enzymatique, une première séparation de charge $S_0 \rightarrow S_1$ a lieu et est suivie par trois autres séparations de charges $S_1 \rightarrow S_2$, $S_2 \rightarrow S_3$, et $S_3 \rightarrow S_4$. A chacune de ces séparations de charge est associée une réaction d'oxydoréduction du cycle de Kok avec, lorsque le cycle est bouclé, formation d'oxygène et de protons libres ainsi que d'électrons libres, et le retour du complexe de l'état $S_4$ vers l'état $S_0$.

En particulier, les auteurs démontrent qu'une des quatre réactions d'oxydoréduction du cycle de Kok : la réaction de réduction, régie par la réactivité du capteur d'électrons, est l'étape limitante du cycle de Kok.

Aussi, Sheleva et Messinger laissent entendre que le rendement du complexe enzymatique PSII pourrait être optimisé en choisissant une valeur de fréquence de pulsion de pulsion prédéterminée qui est une fréquence de pulsion propre de la lumière pulsée associée une vitesse prédéterminée de réaction de réduction qui prend place dans l'environnement immédiat du groupement capteur d'électrons du complexe enzymatique PSII.

De cette façon, à un capteur d'électrons spécifique correspond une fréquence de pulsion spécifique : il s'agit donc d'optimiser la fréquence de pulsion de la lumière pulsée en fonction de la vitesse de réaction de réduction sur le site réducteur du complexe enzymatique PSII et donc en fonction de la nature chimique du capteur d'électrons afin de disposer du nombre le plus élevé possible de turnovers par unité de temps, et donc du nombre de cycles de Kok par unité de temps.

Malheureusement, si le procédé de l'état de l'art avance des conclusions prometteuses en ce qui concerne une voie d'optimisation du fonctionnement du complexe enzymatique PSII, il n'en reste pas moins limité à une contrainte majeure en la nécessité de disposer d'une source de lumière d'énergie constante dans le temps et suffisante pour saturer le cycle de Kok du complexe enzymatique PSII, le niveau d'énergie de la source lumineuse étant régie par la nature physico-chimique du complexe enzymatique PSII, en particulier par la composition chimique des groupements capteurs et donneurs d'électrons.

L'invention a pour but de pallier cet inconvénient de l'état de la technique en procurant un procédé de dissociation d'une solution aqueuse rentable, tout en permettant d'exploiter à l'échelle industrielle une source d'énergie lumineuse est non-contrôlée, comme par exemple l'énergie solaire ou d'origine électrique renouvelable, et la transformer en hydrogène et en oxygène produit *in situ* de façon à pouvoir disposer d'une source de combustible et d'une source de comburant qui pourraient être directement utilisées par exemple sur place.

Pour résoudre ce problème, il est prévu suivant l'invention, un procédé pour dissocier une solution aqueuse, lequel procédé comprend les étapes suivantes :

a) une première oxydation électrochimique de la solution aqueuse en présence de lumière puisée, laquelle lumière pulsée est générée à partir d'une première source d'énergie lumineuse à une valeur de fréquence de pulsion prédéterminée, et ayant une puissance de crête prédéterminée, par une composition enzymatique à base d'un premier complexe enzymatique PSII, isolé d'un deuxième complexe enzymatique PSI, avec production d'oxygène, d'électrons libres et de protons libres dans la solution aqueuse,
b) une capture desdits électrons libres et desdits protons libres, et
c) une capture séparée d'oxygène,

caractérisé en ce que

- ladite énergie lumineuse de ladite première source présente une valeur d'énergie variable dans le temps, ayant une valeur d'énergie lumineuse par seconde déterminée sur une pluralité de pulses de ladite lumière puisée,

et en ce que

- ledit procédé comprend en outre une étape de modulation de ladite valeur de fréquence de pulsion prédéterminée de ladite lumière pulsée à une valeur de fréquence de pulsion suffisante pour obtenir un premier rendement ($\eta_1$) de production d'oxygène par unité d'énergie lumineuse qui soit supérieur d'un facteur (*f*) de 1,01 à 100,00 à un deuxième rendement ($\eta_2$) de production d'oxygène par unité d'énergie lumineuse obtenu pour une deuxième oxydation électrochimique en présence de lumière continue,
- lesdits premier et deuxième rendements ($\eta_i$) étant obtenus chacun à partir d'une vitesse de production d'oxygène ($K(O_2)_i$) par unité d'énergie lumineuse, *i* = 1 ou 2, tels que :

$$\eta_i = \frac{K(O_2)_i}{E_i},$$

où $E_i$ désigne une quantité d'énergie par seconde fournie pour obtenir ladite vitesse de production d'oxygène ($K(O_2)_i$), *I* = 1 ou 2,

- le facteur (f) étant égal à un rapport entre le premier rendement ($\eta_1$) et le deuxième rendement ($\eta_2$) tel que :

$$f = \frac{\eta_1}{\eta_2},$$

lesquelles lumière pulsée et lumière continue présentent une longueur d'onde prédéterminée égale, et la lumière continue présente une puissance égale à la puissance crête prédéterminée de la lumière puisée.

Ainsi, avec le procédé selon la présente invention, pour une irradiation du complexe enzymatique PSII à ladite valeur de fréquence de pulsion suffisante, on assure aisément la production d'oxygène et d'hydrogène qui, en comparaison à une mise en oeuvre du procédé en présence de lumière continue à puissance égale, est caractérisée par un rendement étonnement bien plus élevé.

En effet, bien que ce phénomène ne soit pas encore bien compris, il a été observé de manière tout à fait surprenante qu'en modulant la quantité d'énergie lumineuse variable dans le temps sous forme d'une énergie lumineuse pulsée à une fréquence de pulsion présentant la valeur de fréquence de pulsion suffisante, la vitesse de production d'oxygène lors de l'oxydation de l'eau et donc l'activité du PSII sont augmentées pour une valeur d'énergie totale incidente donnée, comme démontré dans les exemples de la présente demande.

*A contrario*, Sheleva et Messinger, qui utilisent différentes valeurs prédéterminées de fréquence de pulsion pour stimuler le complexe enzymatique, doivent toutefois s'assurer qu'un nombre prédéterminé et constant de pulses soit généré et que chaque pulse présente la même énergie, de sorte que l'énergie totale fournie au système soit constante dans le temps. Aussi, pour une fréquence de pulsion prédéterminée, l'homme de métier qui voudrait mettre en oeuvre ce procédé devrait alors s'assurer que le complexe enzymatique a bien été irradié par le nombre prédéterminé de pulses et doit donc ajuster la durée d'irradiation en fonction de la fréquence de pulsion choisie pour assurer une valeur d'énergie totale incidente constante.

Cet aspect du procédé de l'état de l'art est tout particulièrement contraignant dès lors qu'il s'agit de mettre en place industriellement ce procédé de l'état de la technique par exemple, en tirant profit de la lumière du soleil pour produire de l'hydrogène et de l'oxygène.

A titre illustratif, dans l'exemple du tableau 2 présenté dans les résultats relatifs à la présente invention, la vitesse maximale de production en oxygène par unité d'énergie mesurée en présence de lumière pulsée est au moins 20 fois plus élevée que la vitesse mesurée lors de la mise en oeuvre du procédé d'hydrolyse de l'eau en présence d'une lumière continue, et ce pour une même valeur prédéterminée de puissance lumineuse fixée à 555mW.

En effet, dans le cadre de la présente invention, il a été observé que pour un pulse suffisamment intense et court (de façon à pouvoir saturer le cycle de Kok en lui fournissant l'énergie minimale suffisante pour l'activé et induire la dissociation électrochimique de l'eau), une première séparation de charge ($S_0 \rightarrow S_1$) prend place dans le complexe enzymatique, le nombre de premières séparations de charge est régi par la valeur de fréquence de pulsion suffisante de la lumière puisée.

Le choix de la valeur de fréquence de pulsion suffisante est déterminé par : (i) le profil d'absorption lumineuse du complexe enzymatique et donc par la longueur d'onde de la lumière puisée ; (ii) ainsi que par la valeur d'énergie de l'énergie de la lumière incidente.

Aussi, lorsque la valeur d'énergie de l'énergie lumineuse est plus élevée, la valeur suffisante de fréquence de pulsion est plus élevée, à l'inverse, lorsque la valeur d'énergie de l'énergie lumineuse est plus faible, la valeur suffisante de fréquence de pulsion est plus faible.

Ainsi, le nombre de pulse varie de sorte que la valeur d'énergie de chaque pulse soit suffisante pour créer la

première séparation de charge ($S_0 \rightarrow S_1$) dans le complexe enzymatique. La valeur d'énergie de chaque pulse dépend de la valeur d'énergie de l'énergie lumineuse et du choix de la valeur suffisante de fréquence de pulsion pour obtenir le rendement de production d'oxygène par unité d'énergie lumineuse revendiqué.

Le procédé selon l'invention constitue donc une alternative de mise en oeuvre plus simple que celle du procédé divulgué dans l'état de l'art puisqu'il ne requiert pas le contrôle à la fois de l'énergie appliquée au système et de la fréquence de pulsion avec laquelle cette énergie est appliquée mais seulement de la fréquence de pulsion de la lumière puisée.

Avantageusement, avant ladite étape d'oxydation électrochimique de ladite solution aqueuse, le procédé comprend une étape d'extraction dudit complexe enzymatique PSII de chloroplastes ou de tylakoïdes de chloroplastes.

Dans une forme de réalisation particulière, le procédé selon l'invention comprend, après l'étape d'extraction dudit PSII, une étape de purification dudit complexe enzymatique PSII pour former une composition enzymatique sensiblement concentrée en PSII.

De préférence, lesdits chloroplastes ou tylakoïdes de chloroplastes sont des chloroplastes ou tylakoïdes de chloroplastes de végétaux de la famille des *Chenopodiaceae,* de préférence du genre *Spinacia* ou de la famille des *Characeae,* de préférence des algues procaryotes ou eucaryotes.

Alternativement, ledit complexe PSII est un complexe de synthèse résultant d'une étape de fabrication synthétique dudit complexe enzymatique PSII, ladite étape de fabrication étant réalisée préalablement à l'étape d'oxydation de la solution aqueuse.

Dans une forme de réalisation particulièrement avantageuse du procédé selon l'invention, ladite capture des électrons libres et des protons libres est effectuée par un médiateur de transport d'électrons choisi , par exemple parmi le groupe constitué des dérives de la quinone, de préférence parmi la 2,6-diméthylebenzoquinone, la 2,6-dichloro-p-benzoquinone, et la 1,4-benzoquinone, ou un de leurs mélanges.

Eventuellement, ladite lumière pulsée est émise à une fréquence de pulsion comprise entre 1Hz et 100MHz, de préférence comprise entre 1Hz et 3000Hz, dans une gamme de longueurs d'ondes comprise entre 400nm et 700nm, de préférence entre 500nm et 680nm, à une puissance crête comprise entre 1mW et 800mW, de préférence entre 500 et 700mW.

Dans une forme de réalisation particulière, le procédé selon l'invention est caractérisé en ce que ladite oxydation de l'eau est réalisée à un pH compris entre 4 et 8, de préférence entre 6 et 7.

La mise en oeuvre du procédé selon l'invention dans une gamme de pH comprise entre 6 et 7 assure une activité enzymatique optimale du complexe PSII.

D'autres formes de réalisation du procédé selon l'invention sont indiquées dans les revendications annexées.

L'invention a aussi pour objet une utilisation de lumière puisée, laquelle lumière pulsée générée à partir d'une première source d'énergie lumineuse à une valeur de fréquence de pulsion prédéterminée, et ayant une puissance de crête prédéterminée, pour réaliser une oxydation électrochimique de l'eau en présence de lumière au moyen d'un système biochimique photosensible comprenant :

- une composition enzymatique à base d'un premier complexe enzymatique PSII, isolé d'un deuxième complexe enzymatique PSI, agencé pour oxyder de l'eau en oxygène et pour libérer conjointement des électrons libres et des protons libres dans l'eau,
- un premier capteur desdits électrons libres et un deuxième capteur desdits protons libres, ou un troisième capteur desdits électrons libres et desdits protons libres, qui peut être par exemple une cathode, et
- un quatrième capteur d'oxygène qui peut être par exemple un récipient de stockage d'oxygène

caractérisée en ce que

- ladite énergie lumineuse présente une valeur d'énergie variable dans le temps , ayant une valeur d'énergie lumineuse par seconde déterminée sur une pluralité de pulses de ladite lumière puisée,

et en ce que

- ladite valeur de fréquence de pulsion prédéterminée de ladite lumière pulsée est modulée à une valeur de fréquence de pulsion suffisante pour obtenir un premier rendement ($\eta_1$) de production d'oxygène par unité d'énergie lumineuse qui soit supérieur d'un facteur (*f*) au moins égal de 1,01 à 100,00 à un deuxième rendement ($\eta_2$) de production d'oxygène par unité d'énergie lumineuse obtenu pour une deuxième oxydation électrochimique en présence de lumière continue,
- lesdits premier et deuxième rendements ($\eta_i$) étant obtenus chacun à partir d'une vitesse de production d'oxygène ($(K(O_2)_i)$) par unité d'énergie lumineuse, $i$ = 1 ou 2, tels que :

$$\eta_i = \frac{K(O_2)_i}{E_i},$$

où $E_i$ désigne une quantité d'énergie par seconde fournie pour obtenir ladite vitesse de production d'oxygène ($K(O_2)_i$), $I$ = 1 ou 2,

- le facteur (f) étant égal à un rapport entre le premier rendement ($\eta_1$) et le deuxième rendement ($\eta_2$) tel que :

$$f = \frac{\eta_1}{\eta_2},$$

lesquelles lumière pulsée et lumière continue présentent une longueur d'onde prédéterminée égale, et la lumière continue présente une puissance égale à la puissance crête prédéterminée de la lumière puisée.

Avantageusement, ledit complexe enzymatique PSII est extrait de chloroplastes ou de tylakoïdes de chloroplastes.

De préférence, ledit complexe enzymatique PSII est purifié pour former une composition enzymatique sensiblement concentrée en PSII.

Dans un mode préférentiel d'utilisation, les chloroplastes ou tylakoïdes de chloroplastes sont des chloroplastes ou tylakoïdes de chloroplastes de végétaux de la famille des *Chenopodiaceae,* de préférence du genre *Spinacia* ou de la famille des *Characeae,* de préférence des algues procaryotes ou eucaryotes.

En particulier, ledit complexe enzymatique PSII est un complexe enzymatique de synthèse.

Alternativement, ledit capteur d'électrons libres et de protons libres est un médiateur de transport d'électrons choisi parmi le groupe constitué, par exemple, des dérives de la quinone, de préférence parmi la 2,6-diméthylebenzoquinone, la 2,6-dichloro-p-benzoquinone, et la 1,4-benzoquinone, ou un de leurs mélanges.

Particulièrement, ledit médiateur est présent dans l'eau à une concentration comprise entre 1$\mu$M et 1M. De préférence, le PSII est déposé sur un substrat. La concentration en PSII est dans ce cas de figure au moins supérieure à 1M, ledit substrat jouant alors le rôle d'anode, siège de l'oxydation de l'eau contenue dans la solution aqueuse.

De préférence ledit médiateur est présent dans l'eau à une concentration comprise entre 10$\mu$M et 1mM et dans laquelle ledit PSII est présent dans l'eau à une concentration comprise entre 1$\mu$g Chl/ml et 1g Chl/ml, de préférence entre 20$\mu$g Chl/ml et 200$\mu$g Chl/ml. Au sens de l'invention, les concentrations en PSII sont traditionnellement exprimées par rapport à la concentration en chlorophylle totale (Chl). Par concentration en chlorophylle totale, il faut entendre au sens de l'invention la concentration en chlorophylles A et B.

Préférentiellement, ladite lumière pulsée est émise à une fréquence de pulsion comprise entre 1Hz et 100MHz, de préférence comprise entre 1Hz et 3000Hz, dans une gamme de longueurs d'ondes comprise entre 400nm et 700nm, de préférence entre 500nm et 680nm, à une puissance crête comprise entre 1mW et 800mW, de préférence entre 500 et 700mW.

De manière avantageuse, l'utilisation de lumière pulsée selon l'invention est caractérisée en ce que ladite oxydation de l'eau est réalisée à un pH compris entre 4 et 8, de préférence entre 6 et 7.

D'autres formes de réalisation de l'utilisation de la lumière pulsée suivant l'invention sont indiquées dans les revendications annexées.

La présente invention concerne aussi un dispositif pour mettre en oeuvre le procédé selon l'invention, lequel dispositif comprend :

- une source d'énergie lumineuse destinée à produire une lumière pulsée dont l'énergie lumineuse présente une valeur d'énergie variable dans le temps ;
- un modulateur de fréquence de pulsion de ladite lumière puisée ;
- un réacteur de dissociation d'une solution aqueuse comprenant une phase aqueuse, lequel réacteur est agencé de sorte que ladite solution puisse être irradiée par ladite lumière pulsée lorsque cette dernière est générée ;
- une composition enzymatique en solution dans ladite phase aqueuse, laquelle composition enzymatique comprend un premier complexe PSII, isolé d'une deuxième complexe enzymatique PSI, laquelle composition enzymatique est agencée pour, sous l'action de la lumière puisée, oxyder électrochimique ladite solution aqueuse pour produire de l'oxygène, des électrons libres et des protons libres dans la solution aqueuse ;
- un premier moyen de capture de l'oxygène ;
- un deuxième moyen de capture des électrons libres et des protons libres.

D'autres formes de réalisation du dispositif suivant l'invention sont indiquées dans les revendications annexées.

D'autres caractéristiques et avantages de l'invention ressortiront de la description donnée ci-après, à titre non-limitatif et en faisant référence aux exemples décrits ci-dessous.

La figure 1 illustre les spectres d'absorption du PSII avant et après purification selon une méthode d'extraction du complexe PSII à partir de chloroplastes de l'épinard.
La figure 2 illustre une courbe d'évolution de la vitesse maximale de production d'oxygène obtenue par le procédé selon l'invention en présence de lumière cohérente continue d'une longueur d'onde de 673nm, en fonction de la valeur de l'énergie lumineuse par seconde.
La figure 3 illustre une courbe d'évolution de la de la vitesse maximale de production d'oxygène obtenue par le procédé selon l'invention en présence de lumière cohérente pulsée à 800Hz (puises de durée de 9,2$\mu$s) d'une longueur d'onde de 673nm, en fonction de la valeur de l'énergie lumineuse par seconde.
La figure 4 illustre les courbes d'évolution de la vitesse maximale de production d'oxygène obtenues par le procédé selon l'invention en présence de lumière cohérente continue, ou de lumière cohérente pulsée à 800Hz (puises de durée de 1,5$\mu$s), d'une longueur d'onde de 673nm, en fonction de la valeur de l'énergie lumineuse par seconde.
La figure 5 illustre les courbes d'évolution de rendement énergétique maximal de production d'oxygène obtenues par le procédé selon l'invention en présence de lumière cohérente continue, ou de lumière cohérente pulsée à 800Hz (puises de durée de 1,5$\mu$s), d'une longueur d'onde de 673nm, en fonction de la valeur de l'énergie lumineuse par seconde.
La figure 6 illustre les courbes d'évolution de rendement énergétique maximal de production d'oxygène obtenues par le procédé selon l'invention en présence de lumière cohérente continue, ou de lumière cohérente pulsée à 800Hz (puises de durée de 1,5$\mu$s), d'une longueur d'onde de 673nm, en fonction de la valeur de l'énergie lumineuse par seconde (l'énergie étant exprimée à l'échelle logarithmique).

Le dispositif employé pour mettre en oeuvre le procédé selon la présente invention, dont les performances sont illustrées dans les exemples qui suivent, comprend :

- une source d'énergie lumineuse destinée à produire une lumière pulsée dont l'énergie lumineuse présente une valeur d'énergie variable dans le temps ;
- un modulateur de fréquence de pulsion de ladite lumière puisée ;
- un réacteur de dissociation d'une solution aqueuse comprenant une phase aqueuse, lequel réacteur est agencé de sorte que ladite solution puisse être irradiée par ladite lumière pulsée lorsque cette dernière est générée ;
- une composition enzymatique en solution dans ladite phase aqueuse, laquelle composition enzymatique comprend un premier complexe PSII, isolé d'une deuxième complexe enzymatique PSI, laquelle composition enzymatique est agencée pour, sous l'action de la lumière puisée, oxyder électrochimique ladite solution aqueuse pour produire de l'oxygène, des électrons libres et des protons libres dans la solution aqueuse ;
- un premier moyen de capture de l'oxygène ;
- un deuxième moyen de capture des électrons libres et des protons libres.

En fonctionnement, la lumière pulsée est générée à une première fréquence de pulsion prédéterminée et la source de lumière pulsée est orientée de façon à ce que la lumière pulsée irradie les photo-enzymes dans la solution aqueuse.

L'irradiation du complexe enzymatique en suspension dans la induit une réaction d'oxydation de l'eau contenue dans le réacteur par l'intermédiaire du complexe enzymatique PSII avec production d'oxygène qui est captée par le premier moyen de capture qui est par exemple une électrode de type *Clark* immergée dans la phase aqueuse, les électrons libres et les protons libres sont quant à eux captés par le deuxième moyen de capture qui est un capteur d'électrons, par exemple de la diméthylebenzoquinone (DMBQ) ou de la 2,5-dichloro-p-benzoquinone (DCBQ).

La valeur d'énergie de l'énergie lumineuse variant avec le temps, le modulateur de fréquence de pulsion de la lumière puisée, laquelle lumière pulsée est associée à cette énergie lumineuse variable, permet lorsqu'il est en fonctionnement de moduler la valeur de fréquence de pulsion prédéterminée de cette lumière pulsée à une valeur de fréquence de pulsion suffisante pour obtenir un premier rendement de production d'oxygène par unité d'énergie lumineuse qui soit supérieur d'un facteur compris entre 1,01 et 100,00 à un deuxième rendement de production d'oxygène par unité d'énergie lumineuse obtenu pour une deuxième oxydation électrochimique en présence de lumière continue, lesquelles lumières pulsées et lumières continues présentent une longueur d'onde prédéterminée égale et une puissance prédéterminée égale.

De préférence, le premier rendement de production d'oxygène par unité d'énergie lumineuse qui soit supérieur d'un facteur compris entre 1,01 et 80,00 à un deuxième rendement de production d'oxygène par unité d'énergie lumineuse obtenu pour une deuxième oxydation électrochimique en présence de lumière continue, lesquelles lumières pulsées et lumières continues présentent une longueur d'onde prédéterminée égale et une puissance prédéterminée égale.

Par les termes « fréquence de pulsion », il faut entendre dans la présente invention la fréquence avec laquelle est modulée la valeur d'énergie de l'énergie lumineuse émise à une longueur d'onde prédéterminée ou dans une gamme de longueurs d'onde prédéterminée.

**Exemple 1** : **préparation de la composition enzymatique à base de PSII**

Comme mentionné précédemment, il y a lieu d'éliminer le complexe PSI qui inhibe l'activité du complexe PSII et donc limite la formation d'oxygène.
Des chloroplastes de l'épinard (*Spinacia oleracea*) sont extraits en accord avec la procédure développée par Barthélemy et co. (Journal of Photochemistry and Photobiology B : Biology, 1997, volume 39, pages 213-218), et ensuite extraits des membranes thylakoïdiennes et dissociés du complexe PSI présent dans ces membranes, le complexe PSII selon la méthode de Berthold et co, voir Febs Letters, 1981, volume 134, numéro 2, pages 231-234.
Par rapport à la procédure de Barthélémy, dans le procédé selon l'invention, on fait mention des modifications suivantes:

- la concentration absolue en *Triton® X 100* est maintenue constante dans l'échantillon d'une solution aqueuse en ajustant la concentration de chlorophylle à 200μg/ml avant d'ajouter 25mg de *Triton® X 100* pour 1mg de chlorophylle à l'échantillon, et
- le second traitement au *Triton® X 100* est exclu et remplacé par un rinçage des particules PSII dans un tampon MES-NaOH 20mM (comprenant 15mM de NaCl et 5mM de $MgCl_2$) à pH = 6,5 et une suspension de ces particules dans un tampon MES-NaOH 20mM (comprenant 15mM de NaCl, 5mM de $MgCl_2$, et 0,5M de sucrose) à pH = 6,5.

Le PSII obtenu par la méthode de Bertold *et co.* modifiée, telle que décrite ci-dessus, est ensuite conservé à -80°C après congélation dans de l'azote liquide à une température de -196°C (77K) pendant une période de 10sec.
Selon la méthode d'extraction modifiée décrite ci-dessus, le PSII de l'épinard est isolé du complexe PSI présent dans le chloroplaste. En effet, comme le montre la figure 1, le pic d'absorption apparaissant à 735nm correspond à celui du complexe PSI tandis que le pic d'absorption à 685nm correspond au complexe PSII, ce pic diminuant d'valeur d'énergie d'un facteur 5 après purification selon la méthode du présent exemple, ce qui correspond à une diminution d'un facteur 5 de la concentration en PSI.

**Exemple comparatif 1** : **mesure de la vitesse maximale de production d'oxygène avec le complexe PSII en lumière continue et en lumière pulsée monochromatique à 673nm**

Un système de photohydrolyse constitué d'une cellule, de volume de 2,5ml, dons les parois sont munies de hublots vitrés, dans laquelle est placée une suspension de PSII dans une solution aqueuse à un pH tampon de 6,5, contenant de la diméthylebenzoquinone (DMBQ) ou de la 2,5-dichloro-p-benzoquinone (DCBQ).
La cellule est illuminée par un faisceau de lumière laser à 673 nm, en mode continu ou en mode pulsé. Les caractéristiques du laser sont reprises dans les tableaux 1a (DMBQ) et 1b (DCBQ).
Par les termes « lumière puisée » ou « mode pulsé », il est entendu au sens de l'invention une lumière intermittente, c'est-à-dire dont la valeur d'énergie prend alternativement une première valeur nulle et une deuxième valeur prédéterminée non-nulle à une fréquence de pulsion prédéterminée, par exemple de 800Hz, ce qui signifie que la valeur d'énergie passe 800 fois par seconde de la valeur nulle à la valeur non-nulle prédéterminée par la puissance de la lumière.

Tableau 1a : Caractéristiques du laser utilisé pour le système PSII-DMBQ

| Paramètres | Spécifications |
|---|---|
| Type de laser | *VCSEL* |
| Longueurs d'onde d'émission laser | 671 nm à 15°C<br>676nm à 35°C |
| Largeur spectrale | < 2nm FWHM |
| Mode d'opération | continu ou pulsé |
| Puissance moyenne | 550mW (mode continu et pulsé) |
| Puissance crête | > 700mW (mode pulsé) |
| Taux de répétition | de 1Hz à 3000Hz (mode pulsé) |
| Durée de l'impulsion | 9,2μs (mode pulsé) |
| Sortie optique | collimatée, linéaire, ou focalisée |

Tableau 1b : Caractéristiques du laser utilisé pour le système PSII-DCBQ

| Paramètres | Spécifications |
|---|---|
| Type de laser | *VCSEL* |
| Longueurs d'onde d'émission laser | 671 nm à 15°C<br>676nm à 35°C |
| Largeur spectrale | < 2nm FWHM |
| Mode d'opération | continu ou pulsé |
| Puissance moyenne | 642mW (mode continu et pulsé) |
| Puissance crête | 555mW (mode pulsé) |
| Taux de répétition | de 1Hz à 5000Hz (mode pulsé) |
| Durée de l'impulsion | 1,5μs (mode pulsé) |
| Sortie optique | collimatée, linéaire, ou focalisée |

La vitesse de l'augmentation de la concentration en oxygène sous illumination a été mesurée à 25°C en utilisant une électrode de type *Clark* dans une solution tampon aqueuse de concentration 25mM MES-NaOH (pH = 6,5) et 1mM de DMBQ.

La figure 2 illustre une courbe d'évolution de la vitesse maximale de production d'oxygène (en μmoles) pour 25μg de chlorophylle (Chl), traités par la méthode selon l'exemple 1, par ml de solution aqueuse et par minute (min). La courbe est obtenue en présence de lumière cohérente continue d'une longueur d'onde de 673nm, en fonction de la valeur de l'énergie lumineuse par seconde (en mJ/sec).

La figure 3 illustre une courbe d'évolution de la vitesse maximale de production d'oxygène (en μmoles) pour 25μg de chlorophylle (Chl), traités par la méthode selon l'exemple 1, par ml de solution aqueuse et par minute (min). La courbe est obtenue en présence de lumière cohérente pulsée à 800Hz d'une longueur d'onde de 673nm, en fonction de la valeur de l'énergie lumineuse par seconde (en mJ/sec).

L'analyse des figures 2 et 3 permet de constater, par exemple, que disposant d'une puissance lumineuse de 500mW, la quantité d'énergie par seconde en lumière continue équivaut à 500mJ/sec. A cette quantité d'énergie en lumière continue correspond une vitesse de production d'oxygène $K(O_2)$ de 7 $10^{-2}$ μmoles/(25μg Chl ml min).

En présence d'une lumière puisée, à une puissance de 500mW, émise à une fréquence de pulsion de 100Hz, c'est-à-dire une énergie de 5mJ/sec, correspond une vitesse de production d'oxygène $K(O_2)$ (par énergie) de 0,01535 μmoles/(25μg Chl ml min) par 500 mJ/sec, soit une augmentation d'un facteur 21,9 du rendement de l'énergie, par rapport à la vitesse mesurée en présence d'une lumière continue. Les résultats de la confirmation expérimentale de cette analyse sont repris tableau 2 ci-dessous.

Tableau 2: comparaison de la vitesse de production d'oxygène en présence de lumière pulsée ou continue, d'une puissance de 555 mW à 673 nm.

| Mode | Energie/sec mJ/sec | $K(O_2)$ μmoles/(25μg Chl ml min) | Rendement [$K(O_2)$/ unité d'énergie]* |
|---|---|---|---|
| Continu | 555 | 0,0846 | $1{,}5210^{-4}$ |
| Pulsé | 5,4 | 0,0174 | $32{,}22\ 10^{-4}$ |
| *μmoles/(25μg Chl ml min mJ) | | | |

L'analyse des figures 4 à 6, et en particulier des figures 5 et 6, permet de démontré que le rendement énergétique en mode pulsé est augmenté d'un facteur allant de 1,03 à 61,75 en fonction de la fréquence de pulsion et donc de la quantité d'énergie par seconde, en comparaison à mise en oeuvre du procédé en présence de lumière continue à puissance égale.

Les résultats de la figures 5 sont reportés dans le tableau 3 suivant :

Tableau 3

| Energie (mJ/s) | Rendement énergétique K (O2) μmoles/(25μg Chl ml min)/mJ | Facteur d'augmentation du rendement** |
|---|---|---|
| 1,10 | 0,32727 | 61,75 |

(suite)

| Energie (mJ/s) | Rendement énergétique K (O2) µmoles/(25µg Chl ml min)/mJ | Facteur d'augmentation du rendement** |
|---|---|---|
| 3,21 | 0,22804 | 43,03 |
| 5,50 | 0,16364 | 30,87 |
| 6,42 | 0,15576 | 29,39 |
| 32,10 | 0,04984 | 9,40 |
| 64,20 | 0,02741 | 5,17 |
| 256,80 | 0,01137 | 2,15 |
| 385,20 | 0,00779 | 1,47 |
| 513,60 | 0,00631 | 1,19 |
| 577,80 | 0,00547 | 1,03 |
| **642** | **0,00530** | **1,00** |
| ** par rapport à la lumière continue à une énergie de 642 mJ/s | | |

Il doit être entendu que la présente invention n'est en aucune façon limitée aux modes de réalisation décrits ci-dessus et que bien des modifications peuvent y être apportées dans le cadre des revendications annexées.

## Revendications

**1.** Procédé pour dissocier une solution aqueuse, lequel procédé comprend les étapes suivantes :

a) une première oxydation électrochimique de la solution aqueuse en présence de lumière puisée, laquelle lumière pulsée est générée à partir d'une première source d'énergie lumineuse à une valeur de fréquence de pulsion prédéterminée, et ayant une puissance de crête prédéterminée, par une composition enzymatique à base d'un premier complexe enzymatique PSII, isolé d'un deuxième complexe enzymatique PSI, avec production d'oxygène, d'électrons libres et de protons libres dans la solution aqueuse,
b) une capture desdits électrons libres et desdits protons libres, et
c) une capture séparée d'oxygène,

**caractérisé en ce que**

- ladite énergie lumineuse de ladite première source présente une valeur d'énergie variable dans le temps, ayant une valeur d'énergie lumineuse par seconde déterminée sur une pluralité de pulses de ladite lumière puisée,

**et en ce que**

- ledit procédé comprend en outre une étape de modulation de ladite valeur de fréquence de pulsion prédéterminée de ladite lumière pulsée à une valeur de fréquence de pulsion suffisante pour obtenir un premier rendement ($\eta_1$) de production d'oxygène par unité d'énergie lumineuse qui soit supérieur d'un facteur (*f*) de 1,01 à 100,00 à un deuxième rendement ($\eta_2$) de production d'oxygène par unité d'énergie lumineuse obtenu pour une deuxième oxydation électrochimique en présence de lumière continue,
- lesdits premier et deuxième rendements ($\eta_i$) étant obtenus chacun à partir d'une vitesse de production d'oxygène ($K(O_2)_i$) par unité d'énergie lumineuse, $i$ = 1 ou 2, tels que :

$$\eta_i = \frac{K(O_2)_i}{E_i},$$

où $E_i$ désigne une quantité d'énergie par seconde fournie pour obtenir ladite vitesse de production d'oxygène ($K(O_2)_i$), $i$ = 1 ou 2,
- le facteur (f) étant égal à un rapport entre le premier rendement ($\eta_1$) et le deuxième rendement ($\eta_2$) tel que :

$$f = \frac{\eta_1}{\eta_2},$$

lesquelles lumière pulsée et lumière continue présentent une longueur d'onde prédéterminée égale, et la lumière continue présente une puissance égale à la puissance crête prédéterminée de la lumière puisée.

**2.** Procédé selon la revendication 1, comprenant, avant ladite oxydation électrochimique de ladite solution aqueuse, une étape d'extraction dudit complexe enzymatique PSII de chloroplastes ou de tylakoïdes de chloroplastes.

**3.** Procédé selon la revendication 2, comprenant, après l'étape d'extraction dudit PSII, une étape de purification dudit complexe enzymatique PSII pour former une composition enzymatique concentrée en PSII.

**4.** Procédé selon la revendication 2 ou 3, dans lequel lesdits chloroplastes ou tylakoïdes de chloroplastes sont des chloroplastes ou tylakoïdes de chloroplastes de végétaux de la famille des *Chenopodiaceae,* de préférence du genre *Spinacia* ou de la famille des *Characeae,* de préférence des algues procaryotes ou eucaryotes.

**5.** Procédé selon la revendication 1, comprenant, avant ladite oxydation électrochimique de ladite solution aqueuse, une étape de fabrication synthétique dudit complexe enzymatique PSII.

**6.** Procédé selon l'une quelconque des revendications 1 à 5, dans lequel ladite capture des électrons libres et des protons libres est effectuée par un médiateur de transport d'électron choisi parmi le groupe constitué des dérives de la quinone, de préférence parmi la 2,6-diméthylebenzoquinone, la 2,6-dichloro-p-benzoquinone, et la 1,4-benzoquinone, ou un de leurs mélanges.

**7.** Procédé selon l'un quelconque des revendications précédentes, **caractérisé en ce que** ladite lumière pulsée est émise à une fréquence de pulsion comprise entre 1Hz et 100MHz, de préférence comprise entre 1Hz et 3000Hz, dans une gamme de longueurs d'ondes comprise entre 400nm et 700nm, de préférence entre 500nm et 680nm, à une puissance crête comprise entre 1mW et 800mW, de préférence entre 500 et 700mW.

**8.** Procédé selon l'un quelconque des revendications précédentes, **caractérisé en ce que** ladite oxydation de l'eau est réalisée à un pH compris entre 4 et 8, de préférence entre 6 et 7.

**9.** Procédé selon l'une quelconque des revendications précédentes **caractérisé en ce que** ladite valeur d'énergie est déterminée sur une pluralité de pulses sur un intervalle de temps de 1 s.

**10.** Utilisation de lumière puisée, laquelle lumière pulsée est générée à partir d'une première source d'énergie lumineuse à une valeur de fréquence de pulsion prédéterminée, et ayant une puissance de crête prédéterminée, pour réaliser une oxydation électrochimique de l'eau en présence de lumière au moyen d'un système biochimique photosensible comprenant :

- une composition enzymatique à base d'un premier complexe enzymatique PSII, isolé d'un deuxième complexe enzymatique PSI, agencé pour oxyder de l'eau en oxygène et pour libérer conjointement des électrons libres et des protons libres dans l'eau,
- un premier capteur desdits électrons libres et un deuxième capteur desdits protons libres, ou un troisième capteur desdits électrons libres et desdits protons libres, et
- un quatrième capteur d'oxygène,

**caractérisée en ce que**

- ladite énergie lumineuse présente une valeur d'énergie variable dans le temps , ayant une valeur d'énergie lumineuse par seconde déterminée sur une pluralité de pulses de ladite lumière puisée,

**et en ce que**

- ladite valeur de fréquence de pulsion prédéterminée de ladite lumière pulsée est modulée à une valeur de fréquence de pulsion suffisante pour obtenir un premier rendement ($\eta_1$) de production d'oxygène par unité d'énergie lumineuse qui soit supérieur d'un facteur (*f*) au moins égal de 1,01 à 100,00 à un deuxième rendement ($\eta_2$) de production d'oxygène par unité d'énergie lumineuse obtenu pour une deuxième oxydation électrochimique en présence de lumière continue,
- lesdits premier et deuxième rendements ($\eta_i$) étant obtenus chacun à partir d'une vitesse de production d'oxygène ($K(O_2)_i$) par unité d'énergie lumineuse, *i* = 1 ou 2, tels que :

$$\eta_i = \frac{K(O_2)_i}{E_i},$$

où $E_i$ désigne une quantité d'énergie par seconde fournie pour obtenir ladite vitesse de production d'oxygène ($K(O_2)_i$), / = 1 ou 2,
- le facteur (f) étant égal à un rapport entre le premier rendement ($\eta_1$) et le deuxième rendement ($\eta_2$) tel que :

$$f = \frac{\eta_1}{\eta_2},$$

lesquelles lumière pulsée et lumière continue présentent une longueur d'onde prédéterminée égale, et la lumière continue présente une puissance égale à la puissance crête prédéterminée de la lumière puisée.

**11.** Utilisation de lumière pulsée selon la revendication précédente, dans laquelle ledit complexe enzymatique PSII est extrait de chloroplastes ou de tylakoïdes de chloroplastes.

**12.** Utilisation de lumière pulsée selon la revendication précédente, dans laquelle ledit complexe enzymatique PSII est purifié pour former une composition enzymatique concentrée en PSII.

**13.** Utilisation de lumière pulsée selon la revendication 11 ou 12, dans laquelle lesdits chloroplastes ou tylakoïdes de chloroplastes sont des chloroplastes ou tylakoïdes de chloroplastes de végétaux de la famille des *Chenopodiaceae,* de préférence du genre *Spinacia* ou de la famille des *Characeae,* de préférence des algues procaryotes ou eucaryotes.

**14.** Utilisation de la lumière pulsée selon la revendication 10, dans laquelle ledit complexe enzymatique PSII est un complexe enzymatique de synthèse.

**15.** Utilisation de lumière pulsée selon l'une quelconque des revendications 10 à 14, dans laquelle ledit capteur d'électrons libres et de protons libres est un médiateur de transport d'électrons choisi parmi le groupe constitué des dérives de la quinone, de préférence parmi la 2,6-diméthylebenzoquinone, la 2,6-dichloro-p-benzoquinone, et la 1,4-benzoquinone, ou un de leurs mélanges.

**16.** Utilisation de lumière pulsée selon la revendication 15, dans laquelle ledit médiateur est présent dans l'eau à une concentration comprise entre 1$\mu$M et 1M, de préférence comprise entre 10$\mu$M et 1mM, et dans laquelle ledit PSII est présent dans l'eau à une concentration comprise entre 1$\mu$g Chl/ml et 1g Chl/ml, de préférence entre 20$\mu$g Chl/ml et 200$\mu$g Chl/ml.

**17.** Utilisation de lumière pulsée selon l'un quelconque des revendications 10 à 16, **caractérisé en ce que** ladite lumière pulsée est émise à une fréquence de pulsion comprise entre 1Hz et 100MHz, de préférence comprise entre 1Hz et 3000Hz, dans une gamme de longueurs d'ondes comprise entre 400nm et 700nm, de préférence entre 500nm et 680nm, à une puissance crête comprise entre 1mW et 800mW, de préférence entre 500 et 700mW.

**18.** Utilisation de lumière pulsée selon l'un quelconque des revendications 10 à 17, dans laquelle ladite oxydation de l'eau est réalisée à un pH compris entre 4 et 8, de préférence entre 6 et 7.

**Patentansprüche**

1. Verfahren zur Spaltung einer wässrigen Lösung, wobei das Verfahren die folgenden Schritte umfasst:

    a) eine erste elektrochemische Oxidation der wässrigen Lösung bei Vorhandensein von gepulstem Licht, wobei das gepulste Licht ausgehend von einer ersten Lichtenergiequelle bei einem vorbestimmten Pulsfrequenzwert erzeugt wird, und mit einer vorbestimmten Spitzenleistung, durch eine enzymatische Zusammensetzung auf Basis eines ersten enzymatischen Komplexes PSII, der isoliert ist von einem zweiten enzymatischen Komplex PSI, mit Produktion von Sauerstoff, von freien Elektronen und von freien Protonen in der wässrigen Lösung,
    b) einen Einfang der freien Elektronen und der freien Protonen, und
    c) einen separaten Einfang von Sauerstoff,

    **dadurch gekennzeichnet, dass**

    - die Lichtenergie der ersten Quelle einen in der Zeit variablen Energiewert aufweist, mit einem Lichtenergiewert pro Sekunde, der über eine Vielzahl von Pulsen des gepulsten Lichts bestimmt wird,

    **und dadurch, dass**

    - das Verfahren weiter einen Modulationsschritt des vorbestimmten Pulsfrequenzwerts des gepulsten Lichts auf einen Pulsfrequenzwert umfasst, der ausreichend ist, um eine erste Ausbeute ($\eta_1$) an Sauerstoffproduktion pro Einheit Lichtenergie zu erhalten, die um einen Faktor ($f$) von 1,01 bis 100,00 grösser ist als eine zweite Ausbeute ($\eta_2$) an Sauerstoffproduktion pro Einheit Lichtenergie, die für eine zweite elektrochemische Oxidation bei Vorhandensein von Dauerlicht erhalten wird,
    - wobei die erste und zweite Ausbeute ($\eta_i$) jeweils ausgehend von einer Sauerstoffproduktionsgeschwindigkeit ($K(O_2)_i$) pro Einheit Lichtenergie, $i$ = 1 oder 2, erhalten werden gemäß:

    $$\eta_i = \frac{K(O_2)_i}{E_i}$$

    wo $E_i$ eine Energiemenge pro Sekunde bezeichnet, die geliefert wird, um die Sauerstoffproduktionsgeschwindigkeit ($K(O_2)_i$), $i$ = 1 oder 2, zu erhalten,
    - wobei der Faktor (f) gleich einem Verhältnis zwischen der ersten Ausbeute ($\eta_1$) und der zweiten Ausbeute ($\eta_2$) ist gemäß:

    $$f = \frac{\eta_1}{\eta_2}$$

    wobei das gepulste Licht und das Dauerlicht eine gleiche vorbestimmte Wellenlänge aufweisen und das Dauerlicht eine Leistung aufweist, die gleich der vorbestimmten Spitzenleistung des gepulsten Lichts ist.

2. Verfahren nach Anspruch 1, umfassend, vor der elektrochemischen Oxidation der wässrigen Lösung, einen Extraktionsschritt des enzymatischen Komplexes PSII aus Chloroplasten oder aus Chloroplast-Thylakoiden.

3. Verfahren nach Anspruch 2, umfassend, nach dem Extraktionsschritt des PSII, einen Reinigungsschritt des enzymatischen Komplexes PSII, um eine mit PSII konzentrierte enzymatische Zusammensetzung zu bilden.

4. Verfahren nach Anspruch 2 oder 3, wobei die Chloroplasten oder Chloroplast-Thylakoide pflanzliche Chloroplasten oder Chloroplast-Thylakoide der Familie der *Chenopodiaceae,* vorzugsweise der Gattung *Spinacia* oder der Familie der *Characeae,* vorzugsweise prokaryotische oder eukaryotische Algen sind.

5. Verfahren nach Anspruch 1, umfassend, vor der elektrochemischen Oxidation der wässrigen Lösung, einen Schritt einer synthetischen Herstellung des enzymatischen Komplexes PSII.

6. Verfahren nach einem der Ansprüche 1 bis 5, wobei der Einfang der freien Elektronen und der freien Protonen durch

einen Elektronentransportmediator durchgeführt wird, der ausgewählt ist aus der Gruppe, bestehend aus Chinonderivaten, vorzugsweise aus 2,6-Dimethylebenzochinon, 2,6-Dichloro-p-benzochinon, und 1,4-Benzochinon, oder einer ihrer Mischungen.

**7.** Verfahren nach einem der vorstehenden Ansprüche, **dadurch gekennzeichnet, dass** das gepulste Licht bei einer Pulsfrequenz zwischen 1 Hz und 100 MHz, vorzugsweise zwischen 1 Hz und 3000 Hz, in einem Wellenlängenbereich zwischen 400 nm und 700 nm, vorzugsweise zwischen 500 nm und 680 nm, bei einer Spitzenleistung zwischen 1 mW und 800 mW, vorzugsweise zwischen 500 und 700 mW emittiert wird.

**8.** Verfahren nach einem der vorstehenden Ansprüche, **dadurch gekennzeichnet, dass** die Oxidation des Wassers bei einem pH-Wert zwischen 4 und 8, vorzugsweise zwischen 6 und 7 ausgeführt wird.

**9.** Verfahren nach einem der vorstehenden Ansprüche, **dadurch gekennzeichnet, dass** der Energiewert über eine Vielzahl von Pulsen über ein Zeitintervall von 1 s bestimmt wird.

**10.** Verwendung von gepulstem Licht, wobei das gepulste Licht ausgehend von einer ersten Lichtenergiequelle bei einem vorbestimmten Pulsfrequenzwert erzeugt wird, und mit einer vorbestimmten Spitzenleistung, um eine elektrochemische Oxidation des Wassers bei Vorhandensein von Licht mittels eines lichtempfindlichen biochemischen Systems auszuführen, umfassend:

- eine enzymatische Zusammensetzung auf Basis eines ersten enzymatischen Komplexes PSII, der isoliert ist von einem zweiten enzymatischen Komplex PSI, der angeordnet ist, um Wasser zu Sauerstoff zu oxidieren und um gemeinsam freie Elektronen und freie Protonen im Wasser freizusetzen,
- ein erstes Einfangmittel der freien Elektronen und ein zweites Einfangmittel der freien Protonen, oder ein drittes Einfangmittel der freien Elektronen und der freien Protonen, und
- ein viertes Einfangmittel von Sauerstoff,

**dadurch gekennzeichnet, dass**

- die Lichtenergie einen in der Zeit variablen Energiewert aufweist, mit einem Lichtenergiewert pro Sekunde, der über eine Vielzahl von Pulsen des gepulsten Lichts bestimmt wird,

**und dadurch, dass**

- der vorbestimmte Pulsfrequenzwert des gepulsten Lichts auf einen Pulsfrequenzwert moduliert wird, der ausreichend ist, um eine erste Ausbeute ($\eta_1$) an Sauerstoffproduktion pro Einheit Lichtenergie zu erhalten, die um einen Faktor (*f*), der mindestens gleich 1,01 bis 100,00 ist, grösser ist als eine zweite Ausbeute ($\eta_2$) an Sauerstoffproduktion pro Einheit Lichtenergie, die für eine zweite elektrochemische Oxidation bei Vorhandensein von Dauerlicht erhalten wird,
- wobei die erste und zweite Ausbeute ($\eta_i$) jeweils ausgehend von einer Sauerstoffproduktionsgeschwindigkeit ($K(O_2)_i$) pro Einheit Lichtenergie, *i* = 1 oder 2, erhalten werden gemäß:

$$\eta_i = \frac{K(O_2)_i}{E_i}$$

wo $E_i$ eine Energiemenge pro Sekunde bezeichnet, die geliefert wird, um die Sauerstoffproduktionsgeschwindigkeit ($K(O_2)_i$), *i* = 1 oder 2, zu erhalten,
- wobei der Faktor (f) gleich einem Verhältnis zwischen der ersten Ausbeute ($\eta_1$) und der zweiten Ausbeute ($\eta_2$) ist gemäß:

$$f = \frac{\eta_1}{\eta_2}$$

wobei das gepulste Licht und das Dauerlicht eine gleiche vorbestimmte Wellenlänge aufweisen und das Dauerlicht eine Leistung aufweist, die gleich der vorbestimmten Spitzenleistung des gepulsten Lichts ist.

**11.** Verwendung von gepulstem Licht nach dem vorstehenden Anspruch, wobei der enzymatische Komplex PSII aus Chloroplasten oder aus Thylakoid-Chloroplasten extrahiert ist.

**12.** Verwendung von gepulstem Licht nach dem vorstehenden Anspruch, wobei der enzymatische Komplex PSII gereinigt wird, um eine mit PSII konzentrierte enzymatische Zusammensetzung zu bilden.

**13.** Verwendung von gepulstem Licht nach Anspruch 11 oder 12, wobei die Chloroplasten oder Chloroplast-Thylakoide pflanzliche Chloroplasten oder Chloroplast-Thylakoide der Familie der *Chenopodiaceae,* vorzugsweise der Gattung *Spinacia* oder der Familie der *Characeae,* vorzugsweise prokaryotische oder eukaryotische Algen sind.

**14.** Verwendung von gepulstem Licht nach Anspruch 10, wobei der enzymatische Komplex PSII ein synthetischer enzymatischer Komplex ist.

**15.** Verwendung von gepulstem Licht nach einem der Ansprüche 10 bis 14, wobei das Einfangmittel von freien Elektronen und von freien Protonen ein Elektronentransportmediator ist, der ausgewählt ist aus der Gruppe, bestehend aus Chinonderivaten, vorzugsweise aus 2,6-Dimethylebenzochinon, 2,6-Dichloro-p-benzochinon, und 1,4-Benzochinon, oder einer ihrer Mischungen.

**16.** Verwendung von gepulstem Licht nach Anspruch 15, wobei der Mediator im Wasser bei einer Konzentration zwischen 1 μM und 1 M, vorzugsweise zwischen 10 μM und 1 mM, vorhanden ist und wobei das PSII im Wasser bei einer Konzentration zwischen 1 μg Chl/ml und 1 g Chl/ml, vorzugsweise zwischen 20 μg Chl/ml und 200 μg Chl/ml vorhanden ist.

**17.** Verwendung von gepulstem Licht nach einem der Ansprüche 10 bis 16, **dadurch gekennzeichnet, dass** das gepulste Licht bei einer Pulsfrequenz zwischen 1 Hz und 100 MHz, vorzugsweise zwischen 1 Hz und 3000 Hz, in einem Wellenlängenbereich zwischen 400 nm und 700 nm, vorzugsweise zwischen 500 nm und 680 nm, bei einer Spitzenleistung zwischen 1 mW und 800 mW, vorzugsweise zwischen 500 und 700 mW emittiert wird.

**18.** Verwendung von gepulstem Licht nach einem der Ansprüche 10 bis 17, wobei die Oxidation des Wassers bei einem pH-Wert zwischen 4 und 8, vorzugsweise zwischen 6 und 7 ausgeführt wird.

## Claims

**1.** Method for dissociating an aqueous solution, which method comprises the following steps:

a) a first electrochemical oxidation of the aqueous solution in the presence of pulsed light, which pulsed light is generated from a first light energy source at a predetermined pulse frequency value, and having a predetermined peak power, by an enzymatic composition, based on a first enzymatic complex PSII, isolated from a second enzymatic complex PSI, with production of oxygen, free electrons and free protons in the aqueous solution,
b) a capture of said free electrons and said free protons, and
c) a separate capture of oxygen,

**characterised in that**

- said light energy of said first source has an energy value which is variable over time, having a light energy value per second determined over a plurality of pulses of said pulsed light,

**and in that**

- said method further comprises a step of modulating said predetermined pulse frequency value of said pulsed light at a sufficient pulse frequency to obtain a first yield ($\eta_1$) for producing oxygen per light energy unit which is greater than a factor (*f*) from 1.01 to 100.00 at a second yield ($\eta_2$) for producing oxygen per light energy unit obtained for a second electrochemical oxidation in the presence of continuous light,
- said first and second yields ($\eta_i$) each being obtained from an oxygen production speed ($K(O_2)_i$) per light energy unit, $i$ = 1 or 2, such that:

$$\eta_i = \frac{K(O_2)_i}{E_i}$$

where $E_i$ describes a quantity of energy per second provided to obtain said oxygen production speed ($(K(O_2)_i)$), $i$ = 1 or 2,
- the factor ($f$) being equal to a ratio between the first yield ($\eta_1$) and the second yield ($\eta_2$) such that:

$$f = \frac{\eta_1}{\eta_2}$$

which pulsed light and continuous light have an equal predetermined wavelength, and the continuous light has a power equal to the predetermined peak power of the pulsed light.

**2.** Method according to claim 1, comprising, before said electrochemical oxidation of said aqueous solution, a step of extracting said enzymatic complex PSII from chloroplasts or chloroplast thylakoids.

**3.** Method according to claim 2, comprising, after the step of extracting said PSII, a step of purifying said enzymatic complex PSII to form a PSII-concentrated enzymatic composition.

**4.** Method according to claim 2 or 3, wherein said chloroplasts or chloroplast thylakoids are plant chloroplasts or chloroplast thylakoids of the *Chenopodiaceae* family, preferably of the *Spinacia* type or of the *Characeae* family, preferably prokaryotic or eukaryotic algae.

**5.** Method according to claim 1, comprising, before said electrochemical oxidation of said aqueous solution, a step of synthetically producing said enzymatic complex PSII.

**6.** Method according to any of claims 1 to 5, wherein said capture of free electrons and free protons is achieved by an electron transport mediator selected from among the group consisting of quinone derivatives, preferably from among 2,6-dimethylebenzoquinone, 2,6-dichloro-p-benzoquinone, and 1,4-benzoquinone, or one of the mixtures thereof.

**7.** Method according to any of the preceding claims, **characterised in that** said pulsed light is emitted at a pulse frequency of between 1Hz and 100MHz, preferably between 1Hz and 3000Hz, in a wavelength range of between 400nm and 700nm, preferably between 500nm and 680nm, at a peak power of between 1mW and 800mW, preferably between 500mW and 700mW.

**8.** Method according to any of the preceding claims, **characterised in that** said oxidation of water is achieved at a pH of between 4 and 8, preferably between 6 and 7.

**9.** Method according to any of the preceding claims, **characterised in that** said energy value is determined over a plurality of pulses over a time interval of 1s.

**10.** Use of pulsed light, which pulsed light is generated from a first light energy source at a predetermined pulse frequency value, and having a predetermined peak power, to achieve an electrochemical oxidation of water in the presence of light by means of a photosensitive biochemical system comprising:

- an enzymatic composition based on a first enzymatic complex PSII, isolated from a second enzymatic complex PSI, arranged to oxidise the water with oxygen and to simultaneously release the free electrons and the free protons in the water,
- a first sensor of said free electrons and a second sensor of said free protons, or a third sensor of said free electrons and of said free protons, and
- a fourth oxygen sensor,

**characterised in that**

- said light energy has an energy value which is variable over time, having a light energy value per second

determined over a plurality of pulses of said pulsed light,

**and in that**

- said predetermined pulse frequency value of said pulsed light is modulated at a sufficient pulse frequency to obtain a first yield ($\eta_1$) for producing oxygen per light energy unit which is greater than a factor (*f*) at least equal to 1.01 to 100.00 at a second yield ($\eta_2$) for producing oxygen per light energy unit obtained for a second electrochemical oxidation in the presence of continuous light,
- said first and second yields ($\eta_i$) each being obtained from an oxygen production speed ($K(O_2)_i$) per light energy unit, $i$ = 1 or 2, such that:

$$\eta_i = \frac{K(O_2)_i}{E_i}$$

where $E_i$ means a quantity of energy per second provided to obtain said oxygen production speed ($K(O_2)_i$), $i$ = 1 or 2,
- the factor (*f*) being equal to a ratio between the first yield ($\eta_1$) and the second yield ($\eta_2$) such that:

$$f = \frac{\eta_1}{\eta_2}$$

which pulsed light and continuous light have an equal predetermined wavelength, and the continuous light has a power equal to the predetermined peak power of the pulsed light.

**11.** Use of pulsed light according to the preceding claim, wherein said enzymatic complex PSII is extracted from chloroplasts or from chloroplast thylakoids.

**12.** Use of pulsed light according to the preceding claim, wherein said enzymatic complex PSII is purified to form a PSII-concentrated enzymatic composition.

**13.** Use of pulsed light according to claim 11 or 12, wherein said chloroplasts or chloroplast thylakoids are plant chloroplasts or chloroplast thylakoids of the *Chenopodiaceae* family, preferably of the *Spinacia* type or of the *Characeae* family, preferably prokaryotic or eukaryotic algae.

**14.** Use of pulsed light according to claim 10, wherein said enzymatic complex PSII is a synthesised enzymatic complex.

**15.** Use of pulsed light according to one of claims 10 to 14, wherein said free electron and free proton sensor is an electron transport mediator selected from among the group consisting of quinone derivatives, preferably from among 2,6-dimethylebenzoquinone, 2,6-dichloro-p-benzoquinone, and 1,4-benzoquinone, or one of the mixtures thereof.

**16.** Use of pulsed light according to claim 15, wherein said mediator is present in water at a concentration of between 1$\mu$M and 1M, preferably of between 10$\mu$M and 1mM, and wherein said PSII is present in water at a concentration of between 1$\mu$g Chl/ml and 1g Chl/ml, preferably between 20$\mu$g Chl/ml and 200$\mu$g Chl/ml.

**17.** Use of pulsed light according to one of claims 10 to 16, **characterised in that** said pulsed light is emitted at a pulse frequency of between 1Hz and 100MHz, preferably between 1Hz and 3000Hz, in a wavelength range of between 400nm and 700nm, preferably between 500nm and 680nm, at a peak power of between 1mW and 800mW, preferably of between 500mW and 700mW.

**18.** Use of pulsed light according to any of claims 10 to 17, wherein said oxidation of water is achieved at a pH of between 4 and 8, preferably between 6 and 7.

Extraction de PSII: Spectre de Fluorescence.

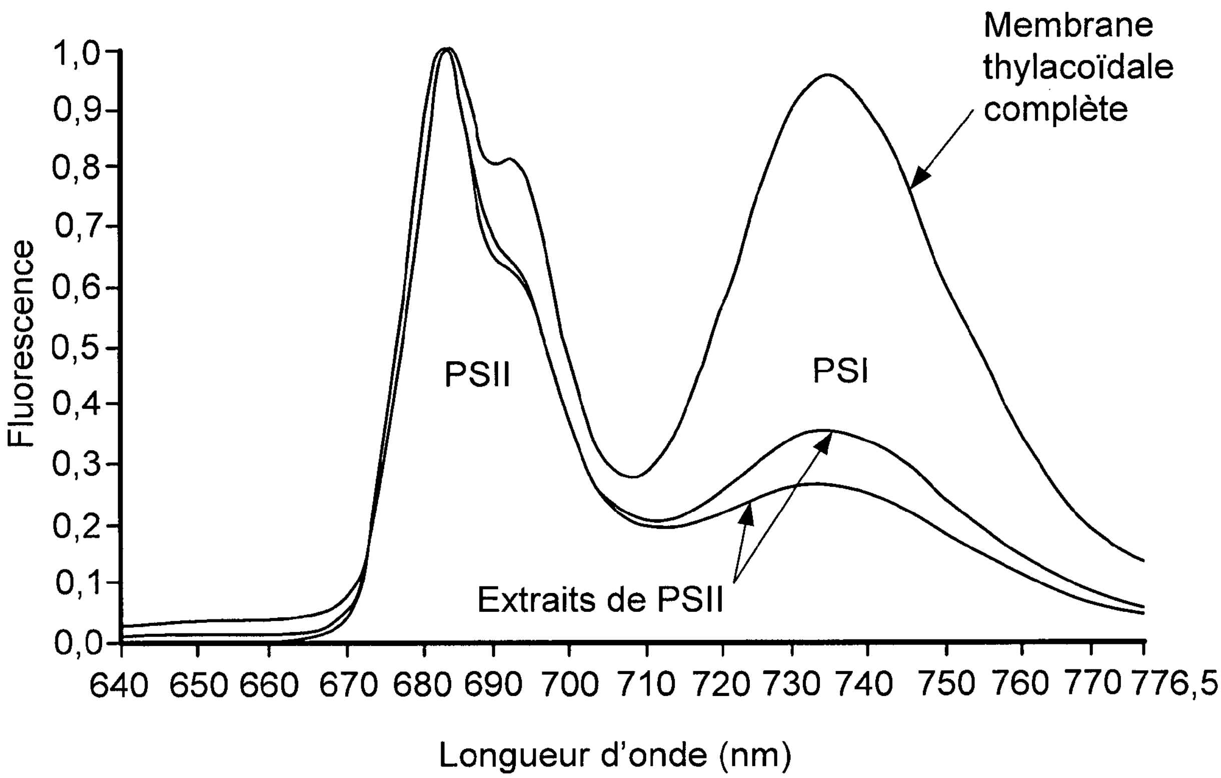


*Fig. 1*

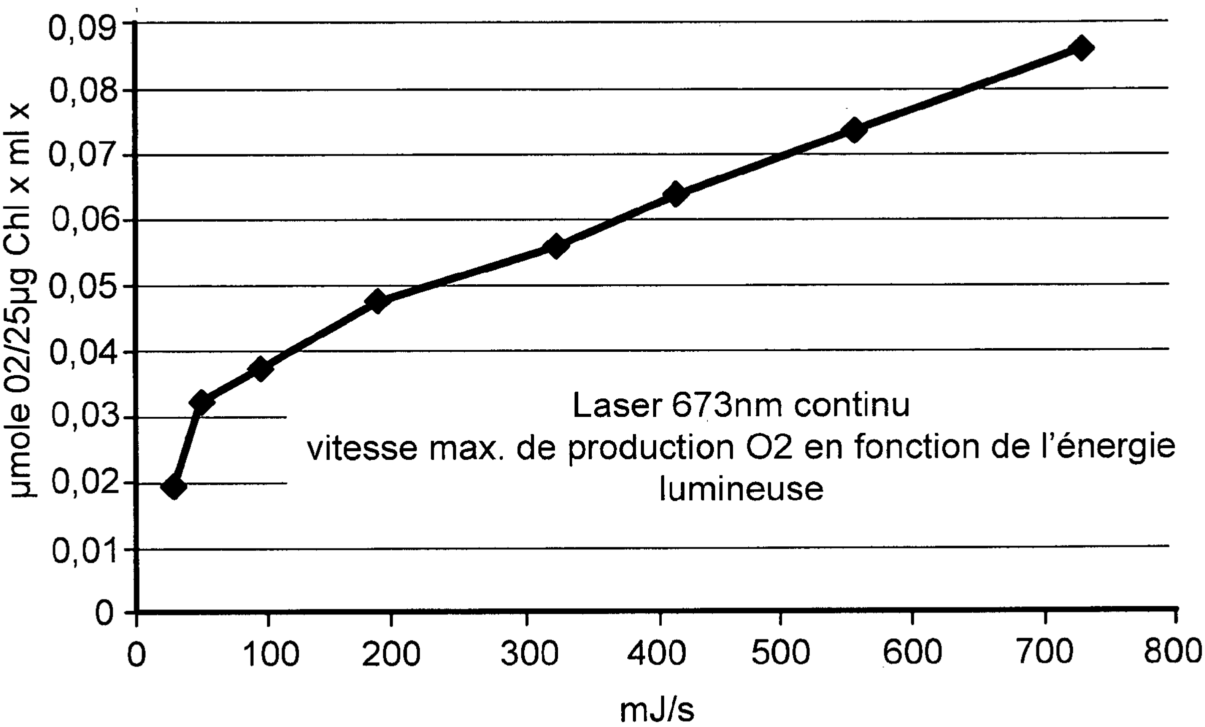
0,09
0,08
0,07
0,06
0,05
0,04
0,03
0,02
0,01
0
µmole 02/25µg Chl x ml x
0
100
200
300
400
500
600
700
800
mJ/s
Laser 673nm continu
vitesse max. de production O2 en fonction de l'énergie
lumineuse

*Fig. 2*

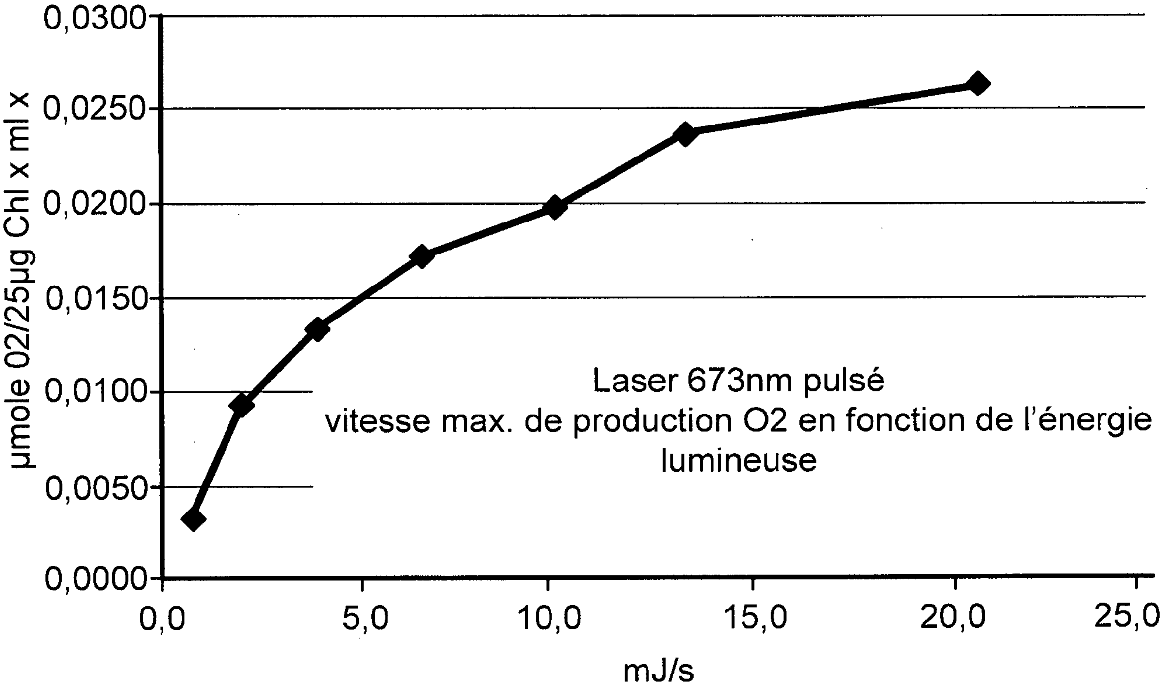
0,0300
0,0250
0,0200
0,0150
0,0100
0,0050
0,0000
µmole 02/25µg Chl x ml x
Laser 673nm pulsé
vitesse max. de production O2 en fonction de l'énergie lumineuse
0,0
5,0
10,0
15,0
20,0
25,0
mJ/s

*Fig. 3*

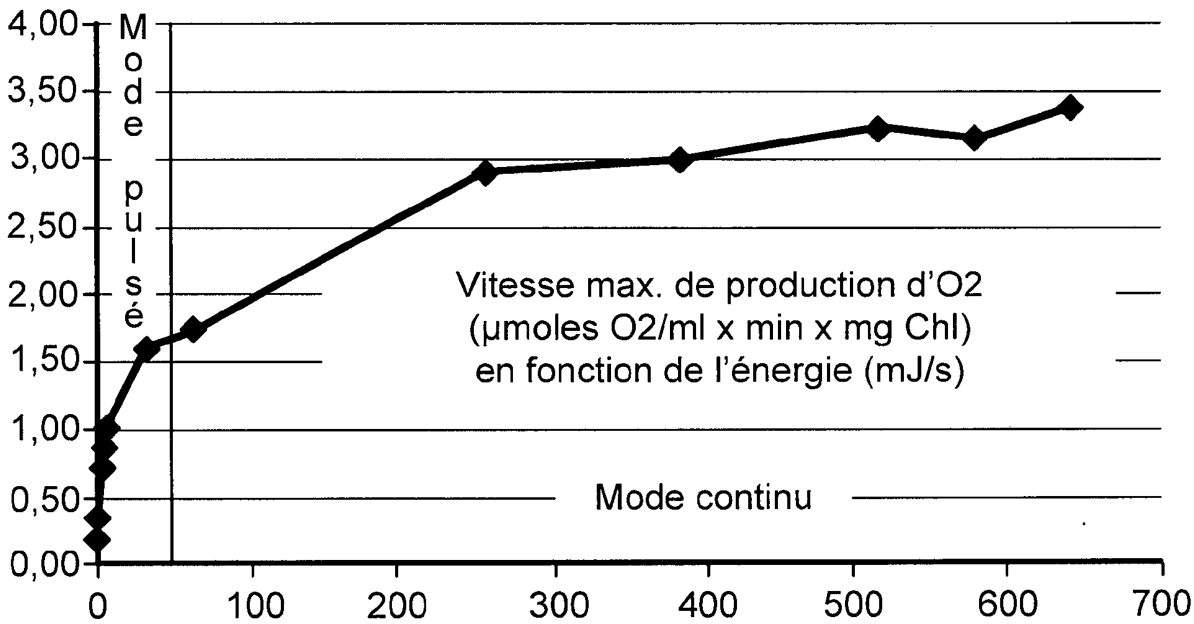
4,00
3,50
3,00
2,50
2,00
1,50
1,00
0,50
0,00
Mode pulsé
Vitesse max. de production d'O2
(µmoles O2/ml x min x mg Chl)
en fonction de l'énergie (mJ/s)
Mode continu
0
100
200
300
400
500
600
700

*Fig. 4*

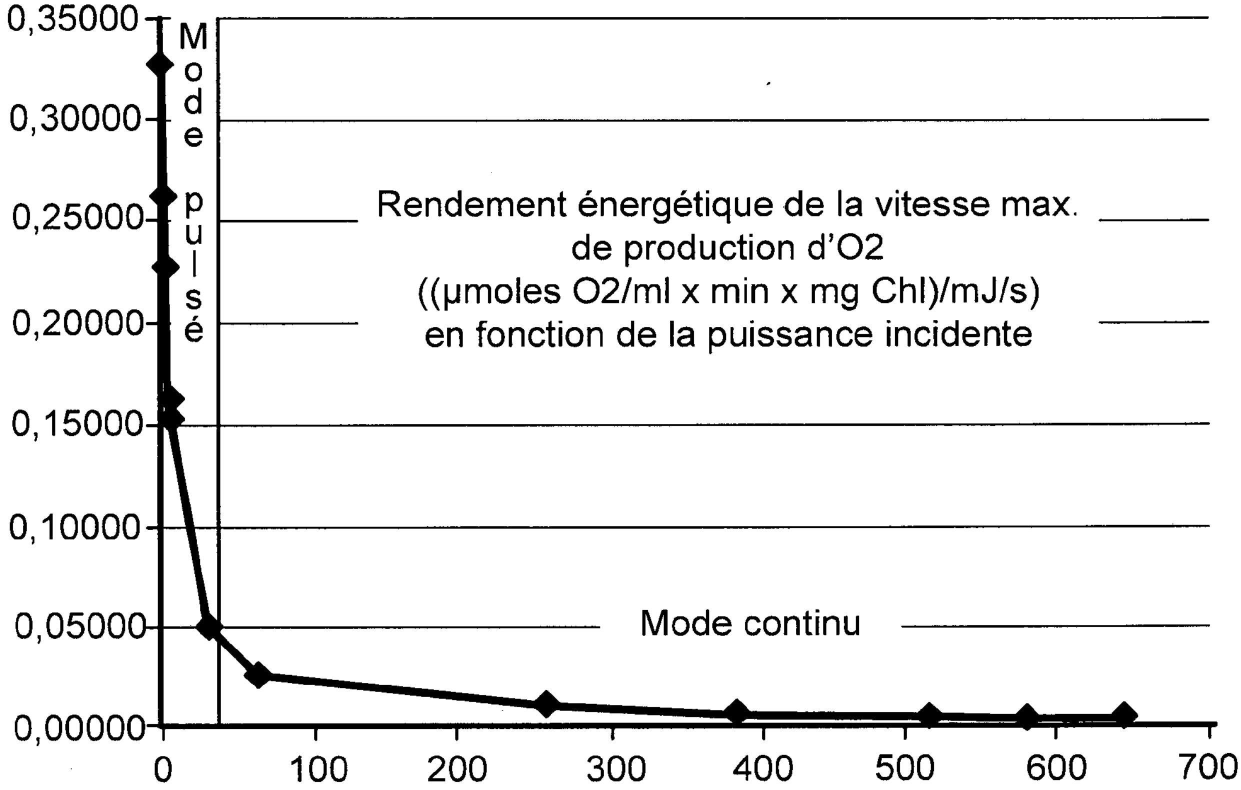

0,35000
0,30000
0,25000
0,20000
0,15000
0,10000
0,05000
0,00000
0
100
200
300
400
500
600
700
Mode pulsé
Rendement énergétique de la vitesse max.
de production d'O2
((µmoles O2/ml x min x mg Chl)/mJ/s)
en fonction de la puissance incidente
Mode continu


***Fig. 5***

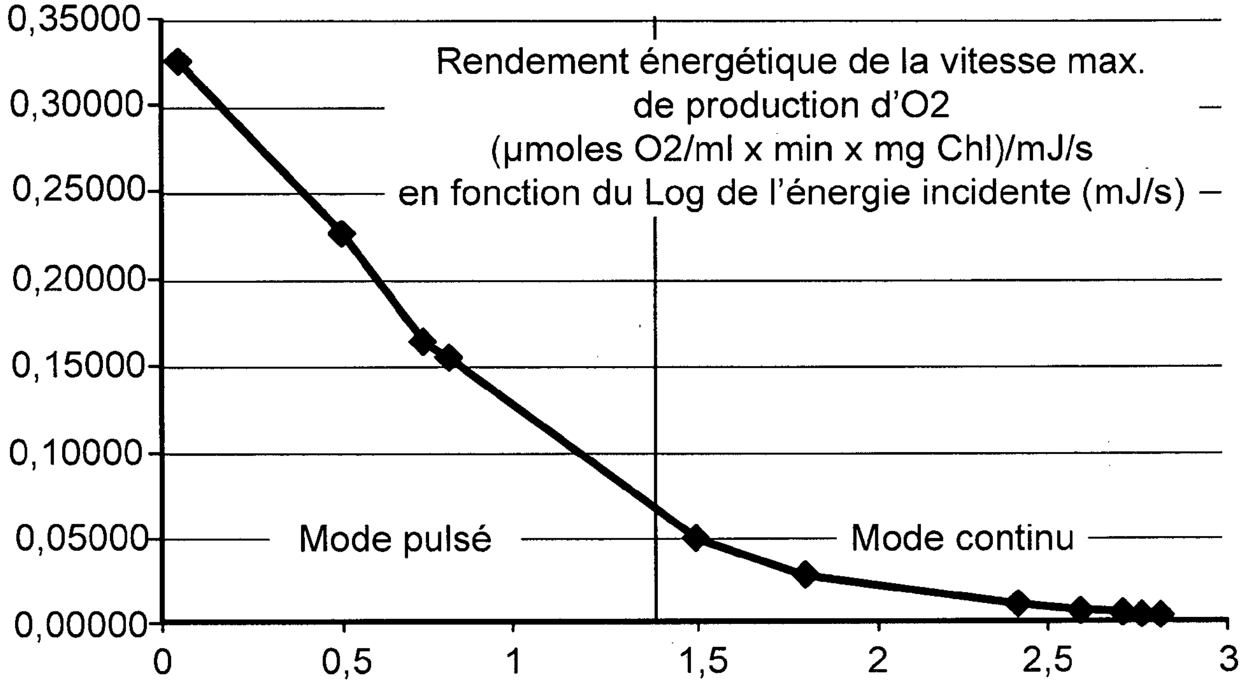
0,35000
0,30000
0,25000
0,20000
0,15000
0,10000
0,05000
0,00000
0
0,5
1
1,5
2
2,5
3
Rendement énergétique de la vitesse max.
de production d'O2
(µmoles O2/ml x min x mg Chl)/mJ/s
en fonction du Log de l'énergie incidente (mJ/s)
Mode pulsé
Mode continu

*Fig. 6*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **SHELEVA ; MESSINGER.** *Biochimica et Biophysica Acta,* 2012, vol. 1817, 1028-1212
- *Biochimica et Biophysica Acta,* 2012, vol. 1817, 1028-1212
- **BARTHÉLEMY.** *Journal of Photochemistry and Photobiology B : Biology,* 1997, vol. 39, 213-218
- **DE BERTHOLD.** *Febs Letters,* 1981, vol. 134 (2), 231-234